(19) Europäisches Patentamt
European Patent Office
Office européen des brevets

(11) Veröffentlichungsnummer: **0 515 941 A1**

(12) **EUROPÄISCHE PATENTANMELDUNG**

(21) Anmeldenummer: **92108332.5**

(22) Anmeldetag: **18.05.92**

(51) Int. Cl.⁵: **C07D 405/04**, C07D 207/34, A01N 43/36, A01N 43/32

(30) Priorität: **30.05.91 DE 4117752**

(43) Veröffentlichungstag der Anmeldung: **02.12.92 Patentblatt 92/49**

(84) Benannte Vertragsstaaten: **BE CH DE ES FR GB IT LI NL PT**

(71) Anmelder: **BAYER AG**

**W-5090 Leverkusen 1 Bayerwerk(DE)**

(72) Erfinder: **Uhr, Hermann, Dr.**
**Düsseldorfer Strasse 67**
**W-5090 Leverkusen 3(DE)**
Erfinder: **Marhold, Albrecht, Dr.**
**Carl-Duisberg-Strasse 329**
**W-5090 Leverkusen 1(DE)**
Erfinder: **Andres, Peter, Dr.**
**Landrat-Trimborn-Strasse 3**
**W-5653 Leichlingen 1(DE)**
Erfinder: **Erdelen, Christoph, Dr.**
**Unterbüscherhof 22**
**W-5653 Leichlingen 3(DE)**
Erfinder: **Wachendorff-Neumann, Ulrike, Dr.**
**Krischerstrasse 81**
**W-4019 Monheim(DE)**
Erfinder: **Stendel, Wilhelm, Dr.**
**In den Birken 55**
**W-5600 Wuppertal 1(DE)**

(54) **Substituierte 2-Arylpyrrole.**

(57) Es werden neue substituierte 2-Arylpyrrole der allgemeinen Formel (I)

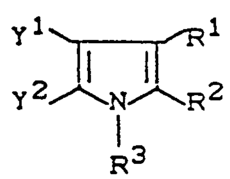

(I)

bereitgestellt,
in welcher

$R^1$ für Cyano oder Nitro steht,
$R^2$ für die Reste

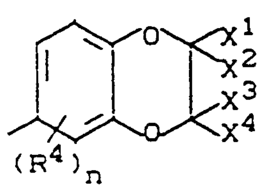 oder 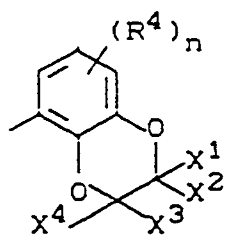 steht,

EP 0 515 941 A1

wobei

| | |
|---|---|
| $X^1$, $X^2$, $X^3$ und $X^4$ | gleich oder verschieden sein können und für Wasserstoff, Halogen oder Alkyl stehen, |
| $R^4$ | für Halogen steht und |
| n | für eine Zahl von 0 bis 3 steht, |
| $R^3$ | für Wasserstoff oder jeweils gegebenenfalls substituiertes Alkyl, Alkenyl oder Alkinyl steht, |
| $Y^1$ | für Halogen und |
| $Y^2$ | für Halogen, Alkyl oder Halogenalkyl steht. |

Die neuen Verbindungen (I) besitzen eine sehr starke Wirksamkeit gegen tierische Schädlinge, insbesondere gegen Insekten, Spinnentiere und Nematoden.

Die vorliegende Erfindung betrifft neue substituierte 2-Arylpyrrole, Zwischenprodukte zu ihrer Herstellung und ihre Verwendung zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen.

Es ist bereits bekannt geworden, daß strukturell ähnliche Cyanopyrrole als Molluskizide, Fungizide und Insektizide wirksam sind (siehe dazu z.B. EP-A 0 347 488, EP-A 0 358 047, EP-A 0 312 723). Die Wirksamkeit und Wirkungsbreite dieser Verbindungen ist jedoch insbesondere bei niedrigen Aufwandmengen und Konzentrationen nicht immer voll zufriedenstellend.

Es wurden nun neue substituierte 2-Arylpyrrole der allgemeinen Formel (I)

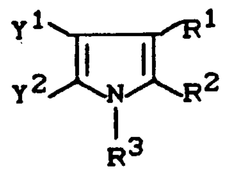

(I)

in welcher

| | |
|---|---|
| $R^1$ | für Cyano oder Nitro steht, |
| $R^2$ | für die Reste |

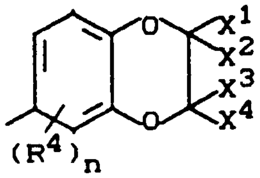 oder 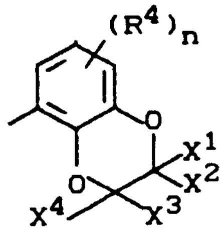 steht,

| | |
|---|---|
| | wobei |
| $X^1$, $X^2$, $X^3$ und $X^4$ | gleich oder verschieden sein können und für Wasserstoff, Halogen oder Alkyl stehen, |
| $R^4$ | für Halogen steht und |
| n | für eine Zahl von 0 bis 3 steht, |
| $R^3$ | für Wasserstoff oder jeweils gegebenenfalls substituiertes Alkyl, Alkenyl oder Alkinyl steht, |
| $Y^1$ | für Halogen und |
| $Y^2$ | für Halogen, Alkyl oder Halogenalkyl steht, |

gefunden.

Weiterhin wurde gefunden, daß man die substituierten 2-Arylpyrrole der allgemeinen Formel (Ia)

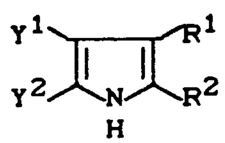

(Ia)

in welcher

$R^1$, $R^2$, $Y^1$ und $Y^2$ die oben angegebene Bedeutung haben, erhält,

wenn man

2-Arylpyrrole der Formel (II)

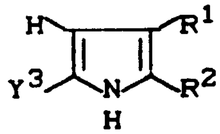

(II)

in welcher
$R^1$ und $R^2$ die oben angegebene Bedeutung haben, und
$Y^3$ für Wasserstoff, Alkyl oder Halogenalkyl steht,
mit Halogenierungsmitteln umsetzt
oder daß man
2-Arylpyrrole der Formel (Ib)

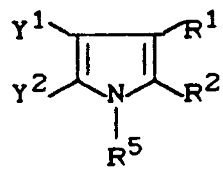

(Ib)

in welcher
$R^1$, $R^2$, $Y^1$ und $Y^2$ die oben angegebene Bedeutung haben und
$R^5$ für jeweils gegebenenfalls substituiertes Alkyl, Alkenyl oder Alkinyl steht, erhält,
wenn man
2-Arylpyrrole der Formel (Ia)

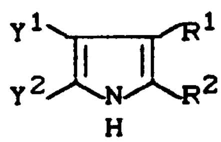

(Ia)

in welcher
$R^1$, $R^2$, $Y^1$ und $Y^2$ die oben angegebene Bedeutung haben, mit Verbindungen der Formel (III)

$R^5$-$X^5$ (III)

in welcher
$R^5$ die oben angegebene Bedeutung hat und
$X^5$ für eine anionische Abgangsgruppe steht,
gegebenenfalls in Gegenwart von Basen und/oder gegebenenfalls in Gegenwart von Verdünnungsmitteln umsetzt.

Schließlich wurde gefunden, daß die neuen substituierten 2-Arylpyrrole der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft in den Forsten, im Vorrats- und Materialschutz sowie auf dem Hygienesektor vorkommen, geeignet sind.

Die erfindungsgemäßen neuen substituierten 2-Arylpyrrole sind durch die allgemeine Formel (I) definiert.

Bevorzugt sind substituierte 2-Arylpyrrole der obigen Formel (I), in welcher
$R^1$ für Cyano oder Nitro steht,
$R^2$ für die Reste

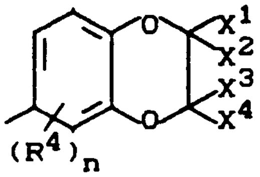 oder 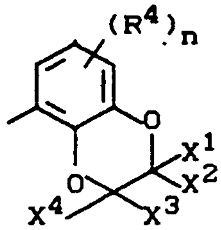 steht,

wobei

| | |
|---|---|
| $X^1$, $X^2$, $X^3$ und $X^4$ | gleich oder verschieden sein können und für Wasserstoff, Halogen oder $C_1$-$C_6$-Alkyl stehen, |
| $R^4$ | für Halogen steht und |
| n | für eine Zahl von 0 bis 3 steht, |
| $R^3$ | für Wasserstoff, $C_1$-$C_6$-Alkyl, $C_3$-$C_6$-Alkenyl oder $C_3$-$C_6$-Alkinyl steht, wobei die Alkyl-, Alkenyl- oder Alkinylreste gegebenenfalls durch 1 bis 4 gleiche oder verschiedene Halogenatome, $C_1$-$C_6$-Alkoxy, $C_1$-$C_6$-Alkylthio, $C_1$-$C_6$-Acyloxy, $C_2$-$C_6$-Alkoxycarbonyl, Phenyl, Cyano oder Nitro substituiert sind, |
| $Y^1$ | für Fluor, Chlor, Brom oder Iod steht und |
| $Y^2$ | für Fluor, Chlor, Brom, Iod, $C_1$-$C_4$-Alkyl oder $C_1$-$C_4$-Halogenalkyl steht. |

Besonders bevorzugt sind substituierte 2-Arylpyrrole der obigen Formel (I), in welcher

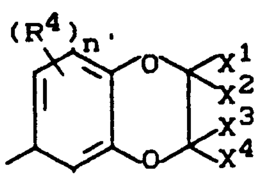 oder 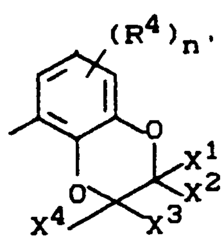 steht,

wobei

| | |
|---|---|
| $X^1$, $X^2$, $X^3$ und $X^4$ | gleich oder verschieden sein können und für Wasserstoff, Fluor, Chlor, Methyl, Ethyl, n-Propyl, i-Propyl stehen, |
| $R^4$ | für Fluor, Chlor oder Brom steht und |
| n' | für eine Zahl von 0 bis 2 steht, |
| $R^3$ | für Wasserstoff, $C_1$-$C_4$-Alkyl, $C_3$-$C_4$-Alkenyl oder $C_3$-$C_4$-Alkinyl steht, wobei die Alkyl-, Alkenyl- oder Alkinylreste gegebenenfalls durch 1 bis 3 gleiche oder verschiedene Halogenatome der Reihe Fluor, Chlor, Brom, $C_1$-$C_4$-Alkoxy, $C_1$-$C_4$-Alkylthio, $C_2$-$C_4$-Acyloxy, $C_2$-$C_4$-Alkoxycarbonyl, Phenyl, Cyano oder Nitro substituiert sind, |
| $Y^1$ | für Chlor oder Brom steht und |
| $Y^2$ | für Chlor, Brom oder $CF_3$ steht. |

Verwendet man gemäß Herstellungsverfahren a) 3-Cyano-2-(2,2,3,3-tetrafluorbenzo-1,4-dioxol-6-yl)-5-trifluormethyl-pyrrol und Brom als Ausgangsstoffe, so kann der Reaktionsverlauf durch das folgende Reaktionsschema wiedergegeben werden:

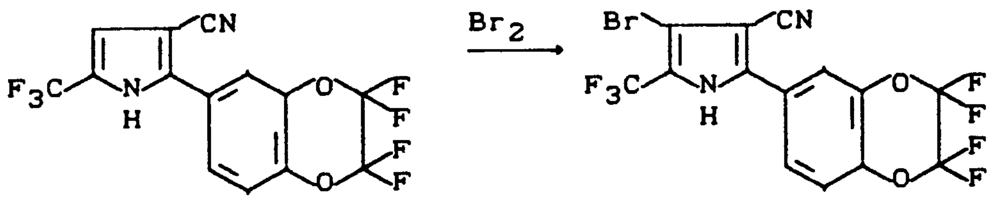


Verwendet man gemäß Herstellungsverfahren a) 3-Cyano-2-(2,2,3,3-tetrafluorbenzo-1,4-dioxol-6-yl)-pyrrol und Sulfurylchlorid als Ausgangsstoffe, so kann der Reaktionsverlauf durch das folgende Reaktionschema wiedergegeben werden:

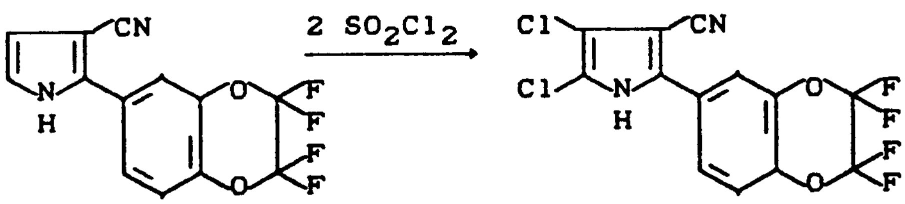


Verwendet man gemäß Verfahren b) 4,5-Dibrom-3-cyano-2-(2,2,3,3-tetrafluorbenzo-1,4-dioxol-6-yl)-pyrrol und Chlormethylethylether als Ausgangsprodukte, so kann der Reaktionsverlauf durch das folgende Reaktionsschema wiedergegeben werden:

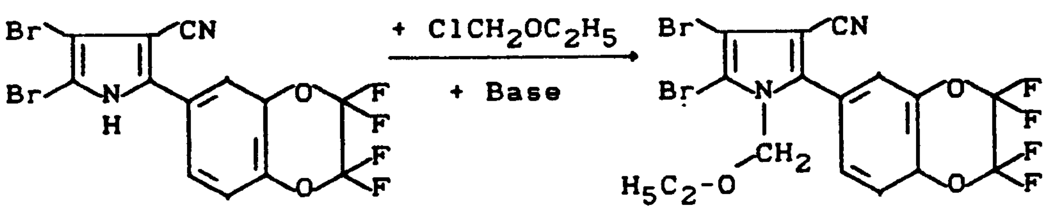


Die beim Herstellungsverfahren a) als Ausgangsstoffe benötigten Verbindungen der allgemeinen Formel (II)

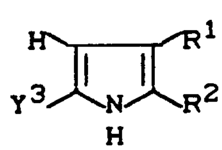

(II)

in welcher
$R^1$, $R^2$ und $Y^3$ die oben angegebene Bedeutung haben, sind neu und lassen sich nach im Prinzip bekannten Methoden herstellen (siehe z.B. I.A. Benages et al, J. Org. Chem. 43, 4278 (1978) oder EP-A 0 347 488).

So erhält man 2-Arylpyrrole der Formel (IIa)

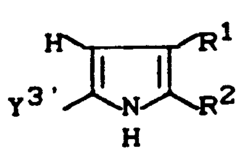

(IIa)

in welcher
$R^1$ und $R^2$ die oben angegebene Bedeutung haben und
$Y^{3'}$ für Alkyl oder Halogenalkyl steht,
indem man
A) Oxazolidine der allgemeinen Formel (IV)

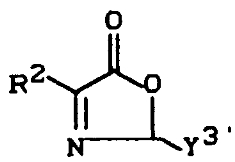

(IV)

in welcher
$R^2$ und $Y^{3'}$ die oben angegebene Bedeutung haben,
mit Verbindungen der allgemeinen Formel (V)

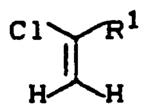

(V)

in welcher
$R^1$ die oben angegebene Bedeutung hat, umsetzt.

Weiterhin erhält man 2-Arylpyrrole der Formel (IIb)

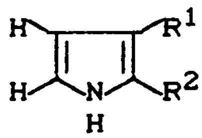

(IIb)

in welcher
$R^1$ und $R^2$ die oben angegebene Bedeutung haben,
entweder
$B_1$) indem man N-Formylarylglycine der allgemeinen Formel (VI)

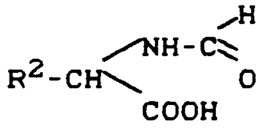

(VI)

in welcher
$R^2$ die oben angegebene Bedeutung hat,
mit Verbindungen der allgemeinen Formel (V)

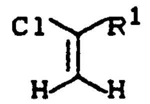

(V)

in welcher

$R^1$ die oben angegebene Bedeutung besitzt, umsetzt

oder

$B_2$) indem man Verbindungen der allgemeinen Formel (VII)

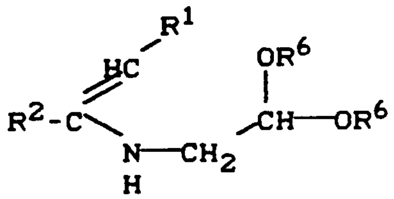

(VII)

in welcher

$R^1$ und $R^2$ die oben angegebene Bedeutung haben und

$R^6$ für Alkyl steht,

mit organischen oder anorganischen Säuren cyclisiert.

Die Herstellung der Ausgangsstoffe der allgemeinen Formel (II) soll durch die folgenden Reaktionsabläufe beispielhaft charakterisiert werden:

Verwendet man bei Variante A) 4-(2,2,3,3-Tetrafluor-benzo-1,4-dioxal-6-yl)-2-(trifluormethyl)-2-oxazolin-5-on und Chloracrylnitril als Ausgangsstoffe, so kann der Reaktionsverlauf durch folgendes Reaktionsschema wiedergegeben werden:

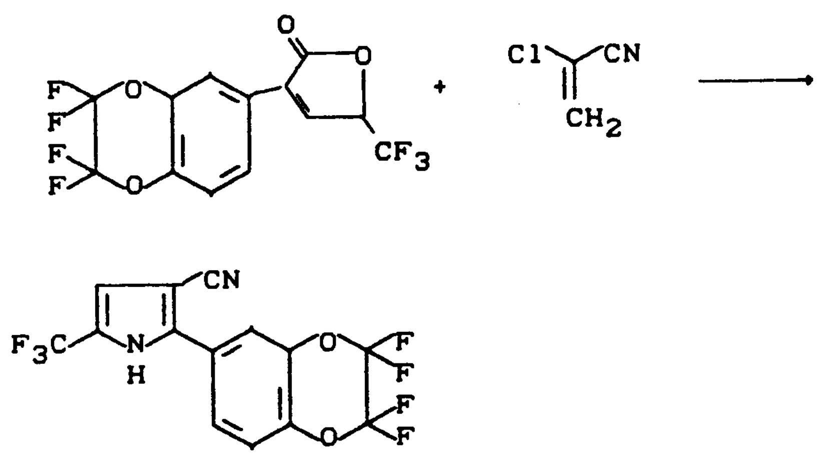

Verwendet man bei Variante $B_1$) N-Formyl-(2,2,3,3-tetrafluor-benzo-1,4-dioxol-6-yl)-glycin, Chloracrylnitril und Acetanhydrid als Ausgangsstoffe, so kann der Reaktionsverlauf durch das folgende Reaktionsschema wiedergegeben werden:

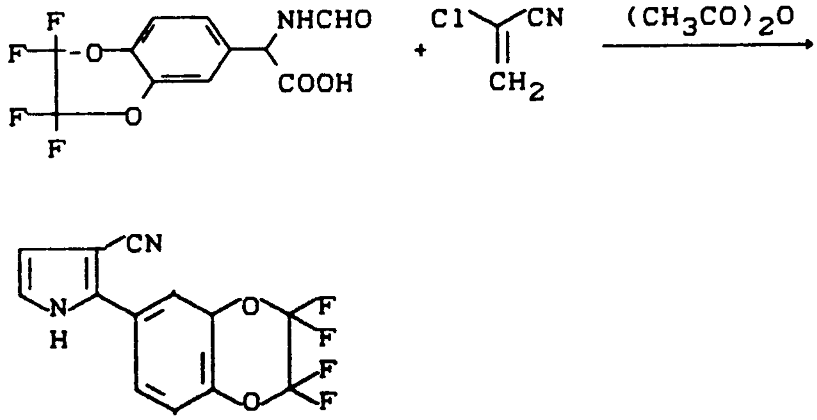


Verwendet man bei Variante $B_2$) untenstehende Verbindung sowie Trifluoressigsäure als Ausgangsstoffe, kann der Reaktionsverlauf durch folgendes Reaktionsschema wiedergegeben werden:

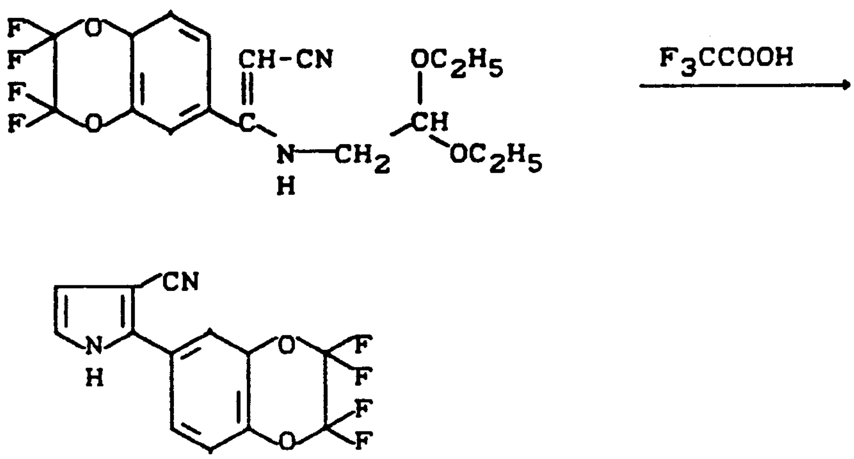


Die bei Verfahren b) zur Herstellung von Verbindungen der Formel (Ib) als Ausgangsstoffe benötigten Verbindungen der allgemeinen Formel (Ia),
in welcher

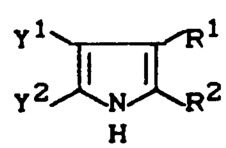
(Ia)

$R^1$, $R^2$, $Y^1$ und $Y^2$ die oben angegebene Bedeutung haben, sind neu und lassen sich nach im Prinzip bekannten Methoden herstellen.

So erhält man Verbindungen der allgemeinen Formel (Ia), wenn man z.B. Verbindungen der allgemeinen Formel (II), in welcher $R^1$, $R^2$ und $Y^3$ die oben angegebene Bedeutung haben mit Verbindungen der Formel (III)

$R^5$-$X^5$ (III)

in welcher

$R^5$ und $X^5$ die oben angegebene Bedeutung haben und gegebenenfalls in Gegenwart von Basen und Verdünnungsmitteln umsetzt.

Das Verfahren a) zur Herstellung von Verbindungen der Formel (Ia) ist dadurch gekennzeichnet, daß Verbindungen der Formel (II), in welcher $R^1$, $R^2$ und $Y^3$ die oben angegebene Bedeutung haben, mit Halogenierungsmitteln behandelt werden. Als Verdünnungsmittel können bei Verfahren a) alle üblichen Lösungsmittel eingesetzt werden. Vorzugsweise verwendbar sind Kohlenwasserstoffe wie Benzol und Hexan, halogenierte Kohlenwasserstoffe wie Chloroform und Methylenchlorid, weiterhin Ether wie Dibutylether, Tetrahydrofuran, Dioxan, außerdem organische Säuren, wie Ameisensäure und Essigsäure.

Weiterhin kann auch Wasser als Lösungsmittel eingesetzt werden. Als Halogenierungsmittel können alle üblichen Halogenierungsmittel eingesetzt werden. Vorzugsweise verwendet man Chlor, Brom, Natriumhypochlorid, Kaliumhypochlorid, Natriumhypobromid, Kaliumhypobromid, Sulfurylchlorid, t-Butylhypochlorid, N-Bromsuccinimid.

Die Temperaturen können innerhalb eines größeren Bereichs variiert werden. Im allgemeinen arbeitet man bei Temperaturen von -10 °C bis +120 °C, vorzugsweise zwischen 0 °C und 70 °C.

Bei der Durchführung setzt man die Reaktionskomponenten der Formel (II) mit äquimolaren oder überschüssigen Mengen eines Halogenierungsmittels um.

Diese Umsetzung wird im allgemeinen unter Normaldruck durchgeführt. Im Falle von Chlor und Brom kann die Reaktion auch bei erhöhtem Druck (bis 5000 hPa) durchgeführt werden.

Das Verfahren b) zur Herstellung von Verbindungen der Formel (Ib) ist dadurch gekenzeichnet, daß man Verbindungen der Formel (Ia) mit Verbindungen der Formel (III), gegebenenfalls in Gegenwart von Basen und gegebenenfalls in Gegenwart von Verdünnungsmitteln umsetzt.

Als Verdünnungsmittel kommen alle inerten organischen Lösungsmittel in Frage. Hierzu gehören vorzugsweise Kohlenwasserstoffe wie Benzol, Toluol, Xylol, ferner Ether, wie Dibutylether, tert.-Butylmethylether, Tetrahydrofuran, Dioxan, außerdem polare Lösungsmittel, wie Dimethylsulfoxid, Acetonitril, Sulfolan, Dimethylformamid und N-Methyl-pyrrolidon.

Als Basen können alle üblichen Protonenakzeptoren eingesetzt werden. Vorzugsweise verwendbar sind Alkalimetall- und Erdalkalimetall-oxide, -hydroxide und -carbonate, wie Natriumyhdroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid oder 18-Krone-6 eingesetzt werden können. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung in einem größeren Bereich variiert werden. Im allgemeinen arbeitet man bei Temperaturen zwischen -10 °C und 200 °C, vorzugsweise zwischen 0 °C und 120 °C.

Bei der Durchführung setzt man die Reaktionskomponenten der Formeln (Ia), die deprotonierenden Basen und die Komponenten der Formeln (III) im allgemeinen in etwa äquimolaren Mengen ein. Es ist jedoch auch möglich, die eine oder andere Komponente in einem größeren Überschuß (bis zu 3 Mol) zu verwenden.

Das Verfahren wird im allgemeinen unter Normaldruck durchgeführt, es kann aber auch unter Druck durchgeführt werden.

Die Herstellung von Vorprodukten der allgemeinen Formel (IIa), in welcher $Y^{3'}$ für Alkyl oder Halogenalkyl steht, ist dadurch gekennzeichnet, daß Oxazolidine der Formel (IV), in welcher $R^2$ die oben angegebene Bedeutung haben mit Verbindungen der allgemeinen Formel (V), wobei $R^1$ die oben angegebene Bedeutung hat, umsetzt. Die Reaktion kann mit oder ohne Verdünnungsmittel durchgeführt werden. Als Verdünnungsmittel können alle üblichen Lösungsmittel eingesetzt werden. Vorzugsweise verwendbar sind Kohlenwasserstoffe wie Benzol und Hexan, halogenierte Kohlenwasserstoffe wie Chloroform und Methylenchlorid, Ether wie Dibutylether, Tetrahydrofuran, Dioxan außerdem Dimethylformamid, Dimethylsulfoxid, Nitromethan.

Die Temperaturen können innerhalb eines größeren Bereichs variiert werden. Im allgemeinen arbeitet man bei Temperaturen von 10 °C bis 200 °C, vorzugsweise zwischen 50 °C und 150 °C. Bei der Durchführung setzt man die Reaktionskomponenten der Formel (IV) mit äquimolaren oder überschüssigen Mengen der Verbindungen (V) um. Bis zu 20-facher Überschuß an (V) ist möglich.

Die Herstellung von Vorprodukten der allgemeinen Formel (IIb) gemäß Verfahrensvariante $B_1$) ist dadurch gekennzeichnet, daß man N-Formylglycine der allgemeinen Formel (VI), in welcher $R^2$ die oben angegebene Bedeutung hat mit Verbindungen der allgemeinen Formel (V), wobei $R^1$ die angegebvene Bedeutung hat, in Gegenwart von Säureanhydriden umsetzt.

Die Reaktion kann mit oder ohne Verdünnungsmittel durchgeführt werden. Als Verdünnungsmittel

können alle üblichen Lösungsmittel eingesetzt werden. Vorzugsweise verwendbar sind Kohlenwasserstoffe wie Benzol und Hexan, halogenierte Kohlenwasserstoffe wie Chloroform und Methylenchlorid, Ether wie Dibutylether, Tetrahydrofuran, Dioxan außerdem Dimethylformamid, Dimethylsulfoxid, Nitromethan.

Die Temperaturen können innerhalb eines größeren Bereichs variiert werden. Im allgemeinen arbeitet man bei Temperaturen von 10 °C bis 200 °C, vorzugsweise zwischen 50 °C und 150 °C. Bei der Durchführung setzt man die Reaktionskomponenten der Formel (VI) mit äquimolaren oder überschüssigen Mengen der Verbindungen (V) um. Bis zu 20-facher Überschuß an (V) ist möglich.

Die Herstellung von Vorprodukten der allgemeinen Formel (IIb) nach Verfahrensvariante $B_2$) ist dadurch gekennzeichnet, daß man Verbindungen der Formel (VII) mit organischen oder anorganischen Säuren cyclisiert. Die Reaktion kann mit oder ohne Verdünnungsmittel durchgeführt werden. Als Verdünnungsmittel können alle üblichen Lösungsmittel verwendet werden. Vorzugsweise verwendbar sind Kohlenwasserstoffe wie Benzol, Toluol oder Hexan, halogenierte Kohlenwasserstoffe wie Chloroform und Methylenchlorid, Ether wie Dibutylether, Tetrahydrofuran, Dioxan, Ketone wie Aceton, außerdem Dimethylformamid, Dimethylsulfoxid, Nitromethan, Wasser oder Mischungen aus den Lösungsmitteln.

Als Säuren können alle üblichen anorganischen und organischen Säuren eingesetzt werden. Vorzugsweise verwendet man Chlorwasserstoffsäure, Bromwasserstoffsäure, Schwefelsäure, Ameisensäure, Essigsäure oder Trifluoressigsäure.

Die Temperaturen können innerhalb eines größeren Bereichs variiert werden. Im allgemeinen arbeitet man bei Temperaturen von -20 °C bis 100 °C, vorzugsweise von -10 °C bis +50 °C.

Bei der Durchführung setzt man die Reaktionskomponenten der Formeln (VII) mit äquimolaren oder überschüssigen Säuremengen, gegebenenfalls in Gegenwart eines Verdünnungsmittels um.

Die erfindungsgemäßen substituierten 2-Arylpyrrole (I) eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen substituierten 2-Arylpyrrole (I) wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge, Flöhe.

Sie sind gegen normalsensible und resistente Arten und Stämme, sowie gegen alle parasitierenden und nicht parasitierenden Entwicklungsstadien der Ektoparasiten wirksam.

Die erfindungsgemäßen substituierten 2-Arylpyrrole (I) zeichnen sich durch eine hohe insektizide Wirksamkeit aus, Sie lassen sich mit gutem Erfolg gegen pflanzenschädigende Insekten, wie beispielsweise gegen die Larven der Meerretttichblattkäfer (Phaedon cochleariae), gegen die grüne Pfirsichblattlaus (Myzus persicae) oder gegen die schwarze Bohnenblattlaus (Aphis fabae) einsetzen. Dabei zeigen die erfindungsgemäßen Wirkstoffe nicht nur protektive sondern auch blattsystemische und wurzelsystemische Eigenschaften.

Daneben eignen sich die erfindungsgemäßen substituierten 2-Arylpyrrole (I) auch zur Bekämpfung von Bodeninsekten und lassen sich beispielsweise zur Bekämpfung der Maden der Zwiebelfliege (Phorbia antiqua) im Boden einsetzen.

Außerdem besitzen die erfindungsgemäßen substituierten 2-Arylpyrrole (I) eine hohe Wirkung gegen Hygiene- und Vorratsschädlinge und lassen sich beispielsweise zur Bekämpfung der deutschen Schabe (Blattella germanica) einsetzen.

Die erfindungsgemäßen substituierten 2-Arylpyrrole (I) können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der erfindungsgemäßen substituierten 2-Arylpyrrole (I) mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Träge stoffe für Granulate kommen in Frage: z.B. gebrochene und fraktionierte natürliche Gesteine wie Calcit, Marmor, Bims, Sepiolith, Dolomit

sowie synthetische Granulate au anorganischen und organischen Mehlen sowie Granulate aus organischem Material wie Sägemehl, Kokosnußschalen, Maiskolben und Tabakstengel; als Emulgier und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synt hetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen substituierten 2-Arylpyrrole (I) können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stofee u.a.

Die erfindungsgemäßen substituierten 2-Arylpyrrole (I) können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß substituierten 2-Arylpyrrole (I) eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen substituierten 2-Arylpyrrole (I) geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Die biologische Wirksamkeit der erfindungsgemäßen Verbindungen soll anhand der Verwendungsbeispiele erläutert werden.

Herstellungsbeispiele

Beispiel 1

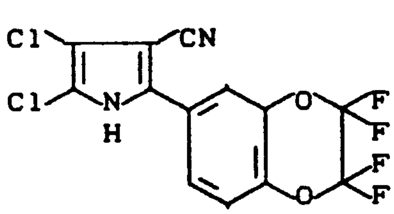

14,7 g (0,049 Mol) 3-Cyano-2-(2,2,3,3-tetrafluorbenzo-1,4-dioxol-6-yl)-pyrrol wurden in 50 ml Eisessig gelöst und innerhalb von 15 Min. mit 8,3 ml Sulfurylchlorid versetzt. Man rührte weitere 15 Min. bei Raumtemperatur, versetzte mit Wasser und saugte ab. Der kristalline Rückstand wurde mit Wasser gewaschen und getrocknet. Ausbeute 17,1 g (94 % d. Th.) der Verbindung obiger Formel (Fp.: = 260 ° C).

Beispiel 2

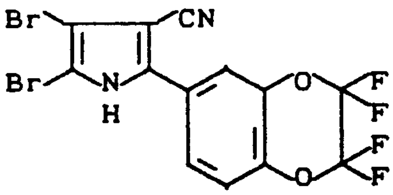

8,4 g (0,027 Mol) 3-Cyano-2-(3-chlor-2,2,3-trifluorbenzo-1,4-dioxol-6-yl)-pyrrol wurden in 200 ml $CHCl_3$ gelöst und nach Zusatz von 8 ml Brom 16 Stunden lang gerührt. Das ausgefallene Reaktionsprodukt wurde abgesaugt, mit viel Petrolether gewaschen und getrocknet. Ausbeute: 8,1 g (64 % d. Th.) der Verbindung obiger Formel.

Beispiel 3

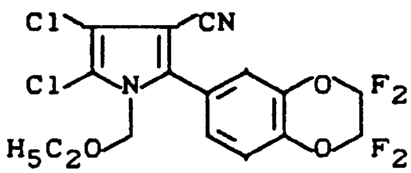

3 g (0,0082 Mol) 4,5-Dichlor-3-cyano-2-(2,2,3,3-tetrafluorbenzo-1,4-dioxol-6-yl)-pyrrol wurden in 50 ml trockenem Tetrahydrofuran gelöst und mit 1 g (0,009 Mol) Kalium-tert.-butylat versetzt. Hierzu tropfte man langsam eine Lösung von 0,78 ml (0,0084 Mol) Chlormethyl-ethylether in Tetrahydrofuran. Es wurde eine Stunde bei Raumtemperatur nachgerührt, auf Wasser gegeben und mit Chloroform extrahiert. Die organische Phase wurde einmal mit Wasser gewaschen, über wasserfreiem Natriumsulfat getrocknet und einrotiert. Ausbeute 3,3 g (95 % d. Th.) der Verbindung obiger Formel (Fp.: = 72 ° C).

In analoger Weise zu Beispiel 1 bis 3 und unter Berücksichtigung der Angaben in der Beschreibung, werden die nachfolgend in Tabelle 1 aufgeführten Substanzen der Formel (I) erhalten.

**Tabelle 1**

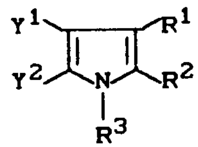

(I)

| Herstellungs-Beispiel Nr. | $R^1$ | $R^2$ | $R^3$ | $Y^1$ | $Y^2$ | physik. Konstanten |
|---|---|---|---|---|---|---|
| 1 | -CN | 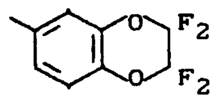 | H | Cl | Cl | Fp. >260° C |
| 2 | -CN | 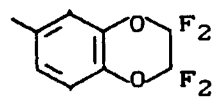 | H | Br | Br | Fp. >260° C |
| 3 | -CN | 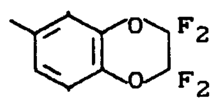 | $-CH_2OC_2H_5$ | Cl | Cl | Fp. = 72° C |
| 4 | -CN | 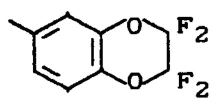 | 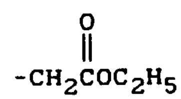 | Cl | Cl | $^1$H-NMR-DMSO δ = 1,12 (3H,t, J = 8 Hz), 4,13 (2H,q, J = 8 Hz), 4,95 (2H, s), 7,4 (1H,dd) 7,6-7,8 (2H,m) |
| 5 | -CN | 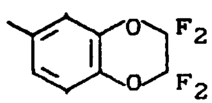 | $-CH_2CH_2-OCH_3$ | Cl | Cl | IR: 2220, 1590 $cm^{-1}$ |

Tabelle 1 - Fortsetzung

| Herstellungs-Beispiel Nr. | $R^1$ | $R^2$ | $R^3$ | $Y^1$ | $Y^2$ | physik. Konstanten |
|---|---|---|---|---|---|---|
| 6 | -CN | 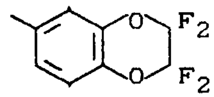 | $CH_3$ | Cl | Cl | $^1$H-NMR (DMSO) δ = 3,58 (3H, s) 7,55 (1H, m) 7,65-7,80 (2H, m) |
| 7 | -CN | 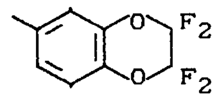 | H | Br | $-CF_3$ | $^1$H-NMR (DMSO) δ = 7.75 (2H, m), 7.90 (1H, m), 13,5 (1H, NH) |
| 8 | -CN | 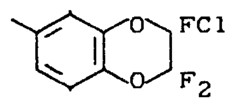 | $-CH_2OC_2H_5$ | Br | $-CF_3$ | $^1$H-NMR (DMSO) δ = 1.21 (3H, t), 2.46 (2H, q), 5.20 (2H, s), 7.25-7.50 (3H, m) |
| 9 | -CN | 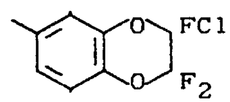 | $-CH_2OC_2H_5$ | Br | Br | Fp. = 66° C |
| 10 | -CN | 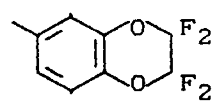 | $-CH_2OC_2H_5$ | Cl | $CF_3$ | |
| 11 | -CN | 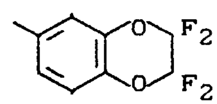 | H | Br | Br | Fp. >260° C |

Tabelle 1 - Fortsetzung

| Herstellungs-Beispiel Nr. | $R^1$ | $R^2$ | $R^3$ | $Y^1$ | $Y^2$ | physik. Konstanten |
|---|---|---|---|---|---|---|
| 12 | -CN | 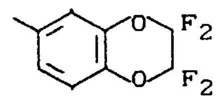 | $-CH_2OC_2H_5$ | Br | Br | $^1H$-NMR ($CDCl_3$) $\delta$ = 1.27 (3H, t), 3.63 (2H, q), 5.20 (2H, s), 7.30-7.50 (3H, m) |
| 13 | $-NO_2$ | 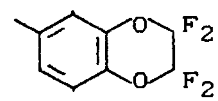 | $-CH_2OC_2H_5$ | Br | Br | |
| 14 | -CN | 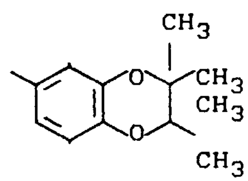 | $-CH_2O-C_2H_5$ | Cl | Cl | |
| 15 | -CN | 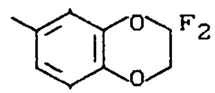 | $-CH_2O-C_3H_7$ | Cl | $CF_3$ | |
| 16 | -CN | 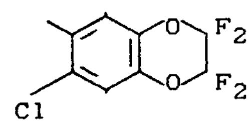 | $-CH_2CN$ | Cl | Cl | |

Tabelle 1 - Fortsetzung

| Herstellungs-Beispiel Nr. | $R^1$ | $R^2$ | $R^3$ | $Y^1$ | $Y^2$ | physik. Konstanten |
|---|---|---|---|---|---|---|
| 17 | -CN | 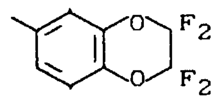 | $-CH_2SC_4H_9$ | Br | Br | |
| 18 | $-NO_2$ | 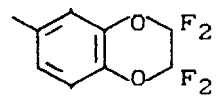 | $-CH_2-CH=CH_2$ | Br | Br | |
| 19 | -CN | 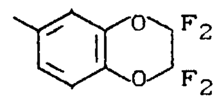 | $-CH_2SCH_3$ | Cl | Cl | |
| 20 | -CN | 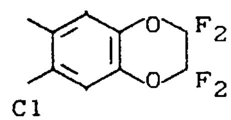 | $-CH_2$ 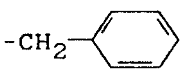 | Cl | $CF_3$ | |
| 21 | -CN | 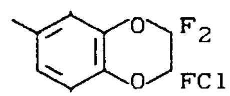 | $-CH_2OC_3H_7$ | Br | Br | $F_p = 108^0 C$ |
| 22 | -CN | 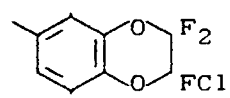 | $-CH_2OC_4H_9$ | Br | Br | $F_p = 144^0 C$ |

Tabelle 1 - Fortsetzung

| Herstellungs-Beispiel Nr. | $R^1$ | $R^2$ | $R^3$ | $Y^1$ | $Y^2$ | physik. Konstanten |
|---|---|---|---|---|---|---|
| 23 | -CN | 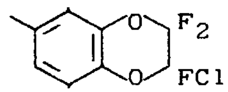 | $-CH_2O$ 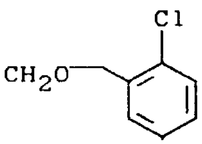 | Br | Br | $F_p$ = 112° C |
| 24 | -CN | 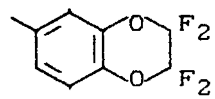 | $-CH_2OC_2H_5$ | Br | $CF_3$ | $^1H$-NMR ($CDCl_3$) δ= 1.20 (3H,t), 3.48 (2H,q), 5.18 (2H,s), 7.25-7.45 (3H,m) |
| 25 | -CN | 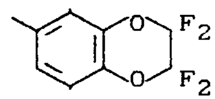 | $-CH_2O-CH(CH_3)_2$ | Br | $CF_3$ | $^1H$-NMR ($CDCl_3$) δ= 1.05 (6H,d), 3.68 (1H,dq), 5.15 (2H,s), 7.25-7.50 (3H,m) |
| 26 | -CN | 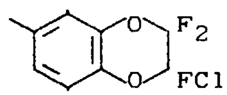 | -H | Br | $CF_3$ | $F_p$ > 200° C |
| 27 | -CN | 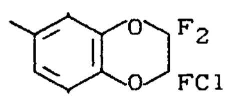 | $-CH_2OCH(CH_3)_2$ | Br | $CF_3$ | $^1H$-NMR ($CDCl_3$) δ= 1.12 (6H,d), 3.65 (1H,dq), 5.20 (2H,s), 7.25-7.50 (3H,m) |

Tabelle 1 - Fortsetzung

| Herstellungs-Beispiel Nr. | $R^1$ | $R^2$ | $R^3$ | $Y^1$ | $Y^2$ | physik. Konstanten |
|---|---|---|---|---|---|---|
| 28 | -CN | 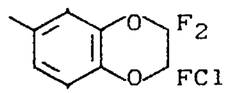 | $-CH_2OC_3H_7$ | Br | $CF_3$ | $^1H$-NMR ($CDCl_3$) δ= 0.90 (3H,t), 1.60 (2H,tq), 3.38 (2H,t), 5.18 (2H,s) 7.25-7.50 (3H,m) |
| 29 | -CN | 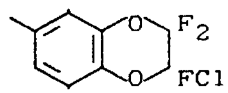 | $-CH_2OCH(CH_2F)_2$ | Br | $CF_3$ | $^1H$-NMR ($CDCl_3$) δ= 3.80-4.10 (1H,m), 4.50 (4H,dd, J=45Hz), 5.38 (2H,1), 7.25-7.50 (3H,m) |
| 30 | -CN | 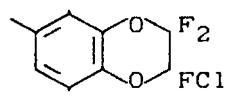 | H | Cl | Cl | $F_p$ = 180° C |
| 31 | -CN | 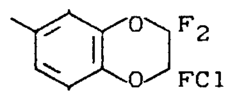 | $-CH_2OC_2H_5$ | Cl | Cl | $F_p$ = 100° C |
| 32 | -CN | 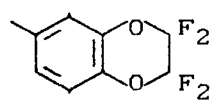 | $-CH_2OC_2H_4-OCH_3$ | Br | Br | $F_p$ = 104° C |

Tabelle 1 - Fortsetzung

| Herstellungs-Beispiel Nr. | $R^1$ | $R^2$ | $R^3$ | $Y^1$ | $Y^2$ | physik. Konstanten |
|---|---|---|---|---|---|---|
| 33 | -CN | 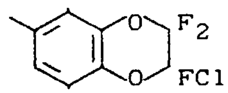 | $-CH_2OCH_2-CH(CH_3)_2$ | Br | Br | $^1H$-NMR ($CDCl_3$) δ= 0.95 (6H,d), 1.88 (1H,dt), 3.30 (2H,d), 5.20 (2H,s) 7.25-7.50 (3H,m) |
| 34 | -CN | 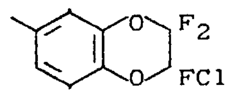 | $-CH_2-O-CH(CH_3)_2$ | Br | Br | $F_p$ = 118° C |
| 35 | -CN | 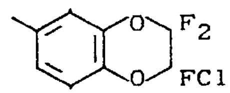 | $-CH_2OC_2H_4OCH_3$ | Br | Br | $F_p$ = 86° C |
| 36 | -CN | 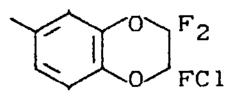 | $-CH_2OCH_2C≡CH$ | Br | Br | $F_p$ = 90° C |
| 37 | -CN | 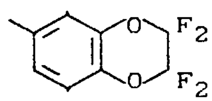 | $-CH_2OC_3H_7$ | Cl | Cl | $^1H$-NMR ($CDCl_3$) δ= 0.95 (3H,t), 1.65 (2H,tq), 3.55 (2H,t), 5.20 (2H,s), 7.25-7.50 (3H,m) |
| 38 | -CN | 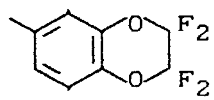 | $-CH_2OCH_2C≡CH$ | Cl | Cl | $^1H$-NMR ($CDCl_3$) δ= 2.42 (1H,t), 4.39 (2H,d), 5.30 (2H,s), 7.25-7.50 (3H,m) |

Verwendungsbeispiele

In den folgenden Verwendungsbeispielen wurde die folgende Verbindung als Vergleichssubstanz eingesetzt:

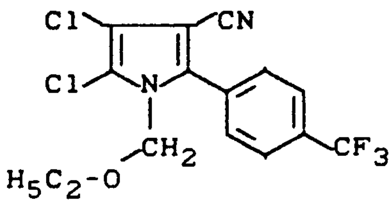

(A)

1-(Ethoxymethyl)-2-(4-trifluormethyl-phenyl)-3-cyano-4,5-dichlor-pyridin bekannt aus: EP-A 0 347 488

Herstellung von Vorprodukten

Beispiel 1A

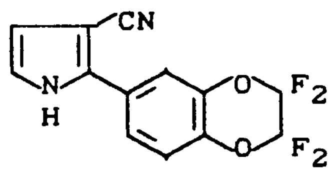

12,5 g (0,032 Mol) des β-Cyanostyrols der Formel

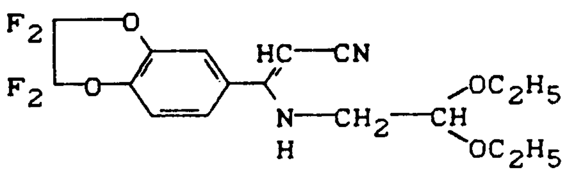

wurden portionsweise in 15 ml Trifluoressigsäure, die auf 0 °C gekühlt war, gegeben. Man rührte eine Stunde lang bei dieser Temperatur nach, saugte das ausgefallene Reaktionsprodukt der obigen erstgenannten Formel ab, wusch mit n-Hexan und trocknete im Vakuum.

Ausbeute: 5,5 g, das entspricht 57 % der Theorie (Fp.: = 210 °C).

In ntsprechender Weise sind die Vorprodukte der folgenden Tabelle 2 erhältlich:

<u>Tabelle 2</u>

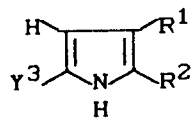

(II)

| Beispiel Nr. | $R^1$ | $R^2$ | $Y^3$ | Verfahrens-variante | physik. Konstanten |
|---|---|---|---|---|---|
| 1A | -CN | 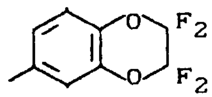 | -H | $B_2$) | Fp.: = 210° C |
| 2A | -CN | 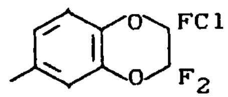 | -H | $B_2$) | Fp.: = 198° C |
| 3A | -CN | 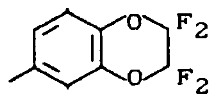 | $-CF_3$ | A) | |
| 4A | $-NO_2$ | 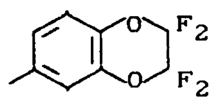 | -H | $B_2$) | |

Tabelle 2 - Fortsetzung

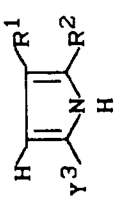

(II)

| Beispiel Nr. | $R^1$ | $R^2$ | $Y^3$ | Verfahrens-variante | physik. Konstanten |
|---|---|---|---|---|---|
| 5A | -CN | 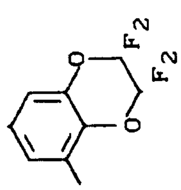 | $-CF_3$ | A) | |
| 6A | -CN | 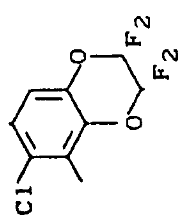 | -H | $B_1$) | |

Beispiel A

Plutella-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat

mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: (3), (5), (6).

Beispiel B

Tetranychus-Test (OP-resistent)

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Entwicklungsstadien der gemeinen Spinnmilbe oder Bohnenspinnmilbe (Tetranychus urticae) befallen sind, werden mit einer Wirkstoffzubereitung der gewünschten Konzentration tropfnaß gespritzt.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

Bei diesem Test zeigt z.B. die folgende Verbindung der Herstellungsbeispiele überlegene Wirksamkeit gegenüber dem Stand der Technik: (3).

Beispiel C

Kornkäfertest

| | |
|---|---|
| Testtiere: | Sitophilus granarius |
| Lösungsmittel: | 35 Gew.-Teile Ethylenglykolmonomethylether |
| | 35 Gew.-Teile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben (ø 9,5 cm) pipettiert, die sich in Petrischalen entsprechender Größe befinden. Nach Trocknung der Filterscheiben werden 30 Testtiere von S. granarius in die Petrischalen überführt und abgedeckt.

Nach 3 Tagen wird die Wirksamkeit der Wirkstoffzubereitung bestimmt. Die Wirksamkeit drückt man in % aus. Dabei bedeutet 100 %, daß alle Kornkafer abgetötet wurden; 0 % bedeutet, daß keine Kornkäfer abgetötet wurden.

Hierbei zeigten z.B. die Verbindungen gemäß den Herstellungsbeispielen (1), (5) und (6) bei einer Wirkstoffkonzentration von 100 ppm eine abtötende Wirkung von 100 %.

Beispiel D

Fliegentest

| | |
|---|---|
| Testtiere: | Musca domestica, Stamm WHO (N) |
| Lösungsmittel: | 35 Gewichtsteile Ethylenglykolmonomethylether |
| | 35 Gewichtsteile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben (ø 9,5 cm) pipettiert, die sich in Petrischalen entsprechender Größe befinden. Nach Trocknung der Filterscheiben werden 25 Testtiere in die Petrischalen überführt und abgedeckt.

Nach 6 Stunden wird die Wirksamkeit der Wirkstoffzubereitung ermittelt. Die Wirksamkeit drückt man in % aus. Dabei bedeutet 100 %, daß alle Fliegen abgetötet wurden; 0 % bedeutet, daß keine Fliegen abgetötet wurden.

Hierbei zeigte z.B. die Verbindung gemäß Herstellungsbeispiel (3) bei einer Wirkstoffkonzentration von 1000 ppm eine abtötende Wirkung von 100 %.

## Patentansprüche

**1.** Substituierte 2-Arylpyrrole der allgemeinen Formel (I)

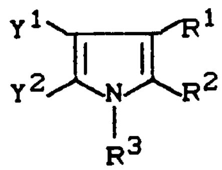

(I)

in welcher

$R^1$ für Cyano oder Nitro steht,

$R^2$ für die Reste

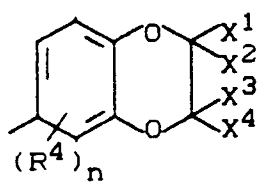

oder

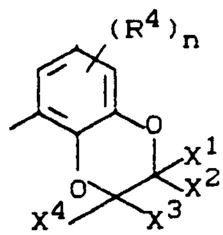

steht,

wobei

$X^1$, $X^2$, $X^3$ und $X^4$ gleich oder verschieden sein können und für Wasserstoff, Halogen oder Alkyl stehen,

$R^4$ für Halogen steht und

n für eine Zahl von 0 bis 3 steht,

$R^3$ für Wasserstoff oder jeweils gegebenenfalls substituiertes Alkyl, Alkenyl oder Alkinyl steht,

$Y^1$ für Halogen und

$Y^2$ für Halogen, Alkyl oder Halogenalkyl steht.

**2.** Substituierte 2-Arylpyrrole der Formel (Ia) gemäß Anspruch 1, in welcher

$R^1$ für Cyano oder Nitro steht,

$R^2$ für die Reste

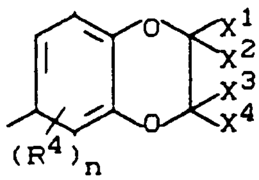 oder 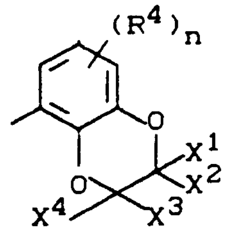 steht,

wobei

$X^1$, $X^2$, $X^3$ und $X^4$ gleich oder verschieden sein können und für Wasserstoff, Halogen oder $C_1$-$C_6$-Alkyl stehen,

$R^4$ für Halogen steht und

n für eine Zahl von 0 bis 3 steht,

$R^3$ für Wasserstoff, $C_1$-$C_6$-Alkyl, $C_3$-$C_6$-Alkenyl oder $C_3$-$C_6$-Alkinyl steht, wobei die Alkyl-, Alkenyl- oder Alkinylreste gegebenenfalls durch 1 bis 4 gleiche oder verschiedene Halogenatome, $C_1$-$C_6$-Alkoxy, $C_1$-$C_6$-Alkylthio, $C_1$-$C_6$-Acyloxy, $C_2$-$C_6$-Alkoxycarbonyl, Phenyl, Cyano oder Nitro substituiert sind,

$Y^1$ für Fluor, Chlor, Brom oder Iod steht und

$Y^2$ für Fluor, Chlor, Brom, Iod, $C_1$-$C_4$-Alkyl oder $C_1$-$C_4$-Halogenalkyl steht.

**3.** Substituierte 2-Arylpyrrole der Formel (1) gemäß Anspruch 1 bis 2, in welcher

$R^1$ für Cyano oder Nitro steht,

$R^2$ für

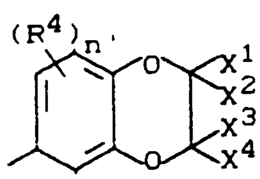 oder 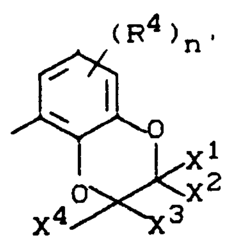 steht,

wobei

$X^1$, $X^2$, $X^3$ und $X^4$ gleich oder verschieden sein können und für Wasserstoff, Fluor, Chlor, Methyl, Ethyl, n-Propyl, i-Propyl stehen,

$R^4$ für Fluor, Chlor oder Brom steht und

n' für eine Zahl von 0 bis 2 steht,

$R^3$ für Wasserstoff, $C_1$-$C_4$-Alkyl, $C_3$-$C_4$-Alkenyl oder $C_3$-$C_4$-Alkinyl steht, wobei die Alkyl-, Alkenyl- oder Alkinylreste gegebenenfalls durch 1 bis 3 gleiche oder verschiedene Halogenatome der Reihe Fluor, Chlor, Brom, $C_1$-$C_4$-Alkoxy, $C_1$-$C_4$-Alkylthio, $C_2$-$C_4$-Acyloxy, $C_2$-$C_4$-Alkoxycarbonyl, Phenyl, Cyano oder Nitro substituiert sind,

$Y^1$ für Chlor oder Brom steht und

$Y^2$ für Chlor, Brom oder $CF_3$ steht.

**4.** Verfahren zur Herstellung von substituierten 2-Arylpyrrolen der allgemeinen Formel (I)

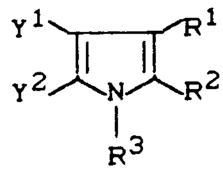

(I)

in welcher

$R^1$ für Cyano oder Nitro steht,

$R^2$ für die Reste

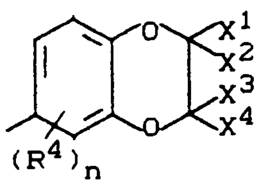

oder

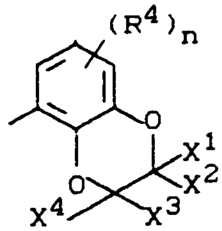

steht,

wobei

$X^1$, $X^2$, $X^3$ und $X^4$ gleich oder verschieden sein können und für Wasserstoff, Halogen oder Alkyl stehen,

$R^4$ für Halogen steht und

n für eine Zahl von 0 bis 3 steht,

$R^3$ für Wasserstoff oder jeweils gegebenenfalls substituiertes Alkyl, Alkenyl oder Alkinyl steht,

$Y^1$ für Halogen und

$Y^2$ für Halogen, Alkyl oder Halogenalkyl steht,

dadurch gekennzeichnet,

a) daß man zum Erhalt von 2-Arylpyrrolen der Formel (Ia)

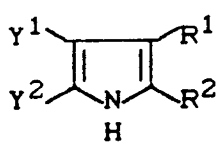

(Ia)

in welcher $R^1$, $R^2$, $Y^1$ und $Y^2$ die oben angegebene Bedeutung haben,

2-Arylpyrrole der Formel (II)

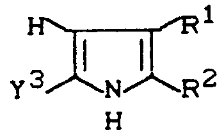

(II)

in welcher

$R^1$ und $R^2$ die oben angegebene Bedeutung haben, und

$Y^3$ für Wasserstoff, Alkyl oder Halogenalkyl steht,

mit Halogenierungsmitteln gegebenenfalls in Gegenwart von Verdünnungsmitteln umsetzt

oder daß man
b) zum Erhalt von 2-Arylpyrrolen der Formel (Ib)

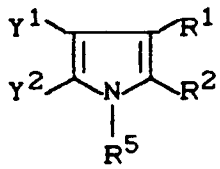

(Ib)

in welcher

$R^1$, $R^2$, $Y^1$ und $Y^2$ die oben angegebene Bedeutung haben und
$R^5$ für jeweils gegebenenfalls substituiertes Alkyl, Alkenyl oder Alkinyl steht,
2-Arylpyrrole der Formel (Ia)

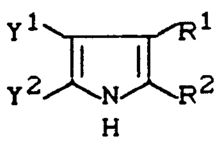

(Ia)

in welcher

$R^1$, $R^2$, $Y^1$ und $Y^2$ die oben angegebene Bedeutung haben,

mit Verbindungen der Formel (III)

$R^5$-$X^5$ (III)

in welcher
$R^5$ die oben angegebene Bedeutung hat und
$X^5$ für eine anionische Abgangsgruppe steht,
gegebenenfalls in Gegenwart von Basen und/oder gegebenenfalls in Gegenwart von Verdünnungsmitteln umsetzt.

**5.** Schädlingsbekämpfungsmittel, gekennzeichnet durch einen Gehalt an mindestens einem substituierten 2-Arylpyrrol der Formel (I) gemäß Anspruch 1 bis 3.

**6.** Verwendung von substituierten 2-Arylpyrrolen der Formel (I) gemäß Anspruch 1 bis 3 zur Bekämpfung von tierischen Schädlingen.

**7.** Verfahren zur Bekämpfung von tierischen Schädlingen, dadurch gekennzeichnet, daß man substituierte 2-Arylpyrrole der Formel (I) gemäß Anspruch 1 bis 3 auf tierische Schädlinge und/oder ihren Lebensraum einwirken läßt.

**8.** Verfahren zur Herstellung von Schädlingsbekämpfungsmitteln, dadurch gekennzeichnet, daß man substituierte 2-Arylpyrrole der Formel (I) gemäß Anspruch 1 bis 5 mit Streckmitteln und/oder oberflächenaktiven Mitteln vermischt.

**9.** Substituierte 2-Arylpyrrole der allgemeinen Formel (II)

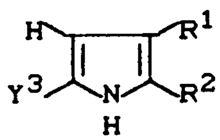

(II)

in welcher

| | |
|---|---|
| $R^1$ | für Cyano oder Nitro steht, |
| $R^2$ | für die Reste |

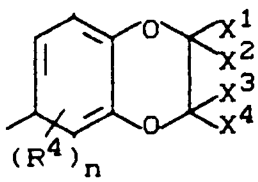

oder

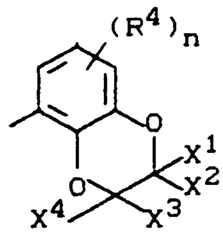

steht,

wobei

| | |
|---|---|
| $X^1$, $X^2$, $X^3$ und $X^4$ | gleich oder verschieden sein können und für Wasserstoff, Halogen oder Alkyl stehen, |
| $R^4$ | für Halogen steht, |
| n | für eine Zahl von 0 bis 3 steht, und |
| $Y^3$ | für Wasserstoff, Alkyl oder Halogenalkyl steht. |

**10.** Verfahren zur Herstellung von substituierten 2-Arylpyrrolen der allgemeinen Formel (II)

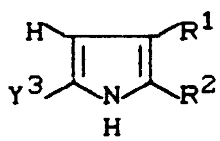

(II)

in welcher

| | |
|---|---|
| $R^1$ | für Cyano oder Nitro steht, |
| $R^2$ | für die Reste |

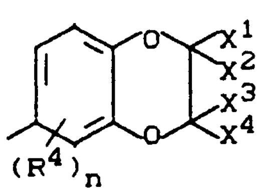

oder

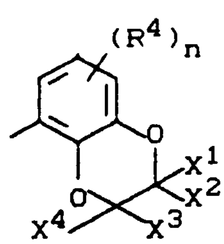

steht,

wobei

| | |
|---|---|
| $X^1$, $X^2$, $X^3$ und $X^4$ | gleich oder verschieden sein können und für Wasserstoff, Halogen oder Alkyl stehen, |
| $R^4$ | für Halogen steht, |

n für eine Zahl von 0 bis 3 steht, und
$Y^3$ für Wasserstoff, Alkyl oder Halogenalkyl steht,
dadurch gekennzeichnet,
A) daß man zum Erhalt von Verbindungen der Formel (IIa)

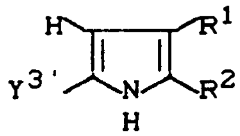

(IIa)

in welcher
$R^1$ und $R^2$ die oben angegebene Bedeutung haben und
$Y^{3'}$ für Alkyl oder Halogenalkyl steht,
Oxazolidine der allgemeinen Formel (IV)

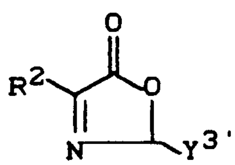

(IV)

in welcher

$R^2$ und $Y^{3'}$ die oben angegebene Bedeutung haben,

mit Verbindungen der allgemeinen Formel (V)

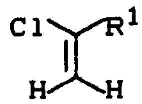

(V)

in welcher
$R^1$ die oben angegebene Bedeutung hat, umsetzt,
B) oder daß man zum Erhalt von 2-Arylpyrrolen der Formel (IIb)

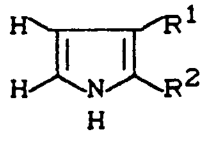

(IIb)

in welcher
$R^1$ und $R^2$ die oben angegebene Bedeutung haben,
entweder
$B_1$) N-Formylarylglycine der allgemeinen Formel (VI)

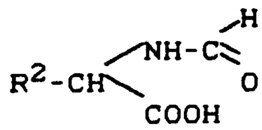

(VI)

in welcher

$R^2$ die oben angegebene Bedeutung hat,

mit Verbindungen der allgemeinen Formel (V)

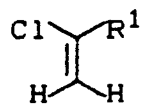

(V)

in welcher

$R^1$ die oben angegebene Bedeutung besitzt, umsetzt

oder

$B_2$) Verbindungen der allgemeinen Formel (VII)

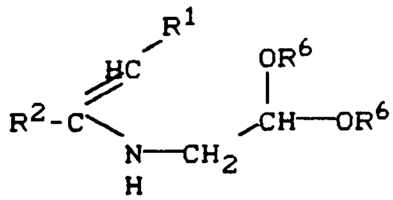

(VII)

in welcher

$R^1$ und $R^2$ die oben angegebene Bedeutung haben und

$R^6$ für Alkyl steht,

mit organischen oder anorganischen Säuren cyclisiert.

**Patentansprüche für folgenden Vertragsstaat : ES**

**1.** Verfahren zur Herstellung von substituierten 2-Arylpyrrolen der allgereinen Formel (I)

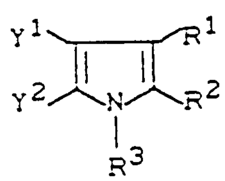

(I)

in welcher

$R^1$ für Cyano oder Nitro steht,

$R^2$ für die Reste

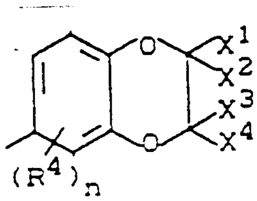 oder 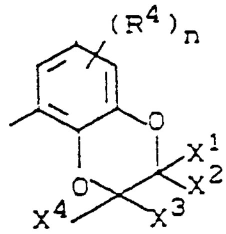 steht,

wobei

| | |
|---|---|
| $X^1$, $X^2$, $X^3$ und $X^4$ | gleich oder verschieden sein können und für Wasserstoff, Halogen oder Alkyl stehen, |
| $R^4$ | für Halogen steht und |
| n | für eine Zahl von 0 bis 3 steht, |
| $R^3$ | für Wasserstoff oder jeweils gegebenenfalls substituiertes Alkyl, Alkenyl oder Alkinyl steht, |
| $Y^1$ | für Halogen und |
| $Y^2$ | für Halogen, Alkyl oder Halogenalkyl steht, |

dadurch gekennzeichnet,

a) daß man zum Erhalt von 2-Arylpyrrolen der Formel (Ia)

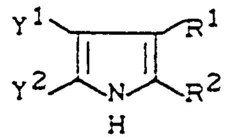

(Ia)

in welcher

$R^1$, $R^2$, $Y^1$ und $Y^2$ die oben angegebene Bedeutung haben,

2-Arylpyrrole der Formel (II)

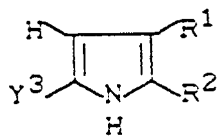

(II)

in welcher

| | |
|---|---|
| $R^1$ und $R^2$ | die oben angegebene Bedeutung haben, und |
| $Y^3$ | für Wasserstoff, Alkyl oder Halogenalkyl steht, |

mit Halogenierungsmitteln gegebenenfalls in Gegenwart von Verdünnungsmitteln umsetzt oder daß man

b) zum Erhalt von 2-Arylpyrrolen der Formel (Ib)

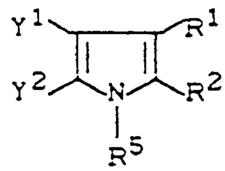

(Ib)

in welcher

$R^1$, $R^2$, $Y^1$ und $Y^2$ die oben angegebene Bedeutung haben und

$R^5$ für jeweils gegebenenfalls substituiertes Alkyl, Alkenyl oder Alkinyl steht,

2-Arylpyrrole der Formel (Ia)

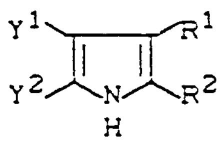

(Ia)

in welcher

$R^1$, $R^2$, $Y^1$ und $Y^2$ die oben angegebene Bedeutung haben,

mit Verbindungen der Formel (III)

$R^5$-$X^5$ (III)

in welcher

$R^5$ die oben angegebene Bedeutung hat und

$X^5$ für eine anionische Abgangsgruppe steht,

gegebenenfalls in Gegenwart von Basen und/oder gegebenenfalls in Gegenwart von Verdünnungsmitteln umsetzt.

**2.** Verfahren gemäß Anspruch 1 zur Herstellung von Verbindungen der Formel (I), in welcher

$R^1$ für Cyano oder Nitro steht,

$R^2$ für die Reste

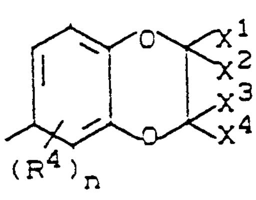

oder

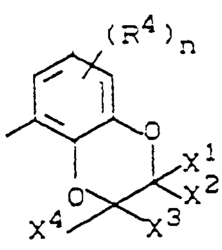

steht,

wobei

$X^1$, $X^2$, $X^3$ und $X^4$ gleich oder verschieden sein können und für Wasserstoff, Halogen oder $C_1$-$C_6$-Alkyl stehen,

$R^4$ für Halogen steht und

n für eine Zahl von 0 bis 3 steht,

$R^3$ für Wasserstoff, $C_1$-$C_6$-Alkyl, $C_3$-$C_6$-Alkenyl oder $C_3$-$C_6$-Alkinyl steht, wobei die

Alkyl-, Alkenyl- oder Alkinylreste gegebenenfalls durch 1 bis 4 gleiche oder verschiedene Halogenatome, $C_1$-$C_6$-Alkoxy, $C_1$-$C_6$-Alkylthio, $C_1$-$C_6$-Acyloxy, $C_2$-$C_6$-Alkoxycarbonyl, Phenyl, Cyano oder Nitro substituiert sind,

$Y^1$ für Fluor, Chlor, Brom oder Iod steht und

$Y^2$ für Fluor, Chlor, Brom, Iod, $C_1$-$C_4$-Alkyl oder $C_1$-$C_4$-Halogenalkyl steht.

**3.** Verfahren gemäß Anspruch 1 zur Herstellung von substituierten 2-Arylpyrrolen der Formel (I) genäß Anspruch 1 bis 2, in welcher

$R^1$ für Cyano oder Nitro steht,

$R^2$ für

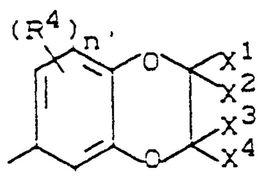

oder

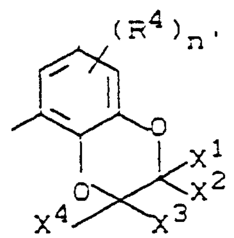

steht,

wobei

$X^1$, $X^2$, $X^3$ und $X^4$ gleich oder verschieden sein können und für Wasserstoff, Fluor, Chlor, Methyl, Ethyl, n-Propyl, i-Propyl stehen,

$R^4$ für Fluor, Chlor oder Brom steht und

n' für eine Zahl von 0 bis 2 steht,

$R^3$ für Wasserstoff, $C_1$-$C_4$-Alkyl, $C_3$-$C_4$-Alkenyl oder $C_3$-$C_4$-Alkinyl steht, wobei die Alkyl-, Alkenyl- oder Alkinylreste gegebenenfalls durch 1 bis 3 gleiche oder verschiedene Halogenatome der Reihe Fluor, Chlor, Brom, $C_1$-$C_4$-Alkoxy, $C_1$-$C_4$-Alkylthio, $C_2$-$C_4$-Acyloxy, $C_2$-$C_4$-Alkoxycarbonyl, Phenyl, Cyano oder Nitro substituiert sind,

$Y^1$ für Chlor oder Brom steht und

$Y^2$ für Chlor, Brom oder $CF_3$ steht.

**4.** Schädlingsbekämpfungsmittel, gekennzeichnet durch einen Gehalt an mindestens einem substituierten 2-Arylpyrrol der Formel (I) gemäß Anspruch 1 bis 3.

**5.** Verwendung von substituierten 2-Arylpyrrolen der Formel (I) gemäß Anspruch 1 bis 3 zur Bekämpfung von tierischen Schädlingen.

**6.** Verfahren zur Bekämpfung von tierischen Schädlingen, dadurch gekennzeichnet, daß man substituierte 2-Arylpyrrole der Formel (I) gemäß Anspruch 1 bis 3 auf tierische Schädlinge und/oder ihren Lebensraum einwirken läßt.

**7.** Verfahren zur Herstellung von Schädlingsbekämpfungsmitteln, dadurch gekennzeichnet, daß man substituierte 2-Arylpyrrole der Formel (I) gemäß Anspruch 1 bis 3 mit Streckmittein und/oder oberflächenaktiven Mitteln vermischt.

**8.** Verfahren zur Herstellung von substituierten 2-Arylpyrrolen der allgemeinen Formel (II)

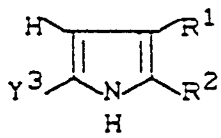

(II)

in welcher

| | |
|---|---|
| $R^1$ | für Cyano oder Nitro steht, |
| $R^2$ | für die Reste |

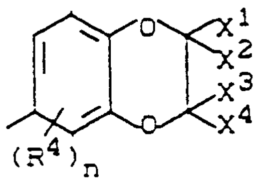

oder

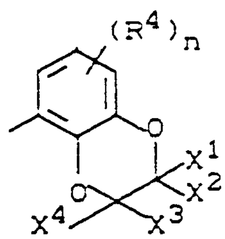

steht,

| | |
|---|---|
| | wobei |
| $X^1$, $X^2$, $X^3$ und $X^4$ | gleich oder verschieden sein können und für Wasserstoff, Halogen oder Alkyl stehen, |
| $R^4$ | für Halogen steht, |
| n | für eine Zahl von 0 bis 3 steht, und |
| $Y^3$ | für Wasserstoff, Alkyl oder Halogenalkyl steht, |

dadurch gekennzeichnet,

A) daß man zum Erhalt von Verbindungen der Formel (IIa)

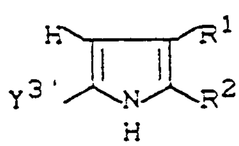

(IIa)

in welcher

| | |
|---|---|
| $R^1$ und $R^2$ | die oben angegebene Bedeutung haben und |
| $Y^{3'}$ | für Alkyl oder Halogenalkyl steht, |

Oxazolidine der allgemeinen Formel (IV)

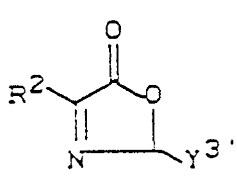

(IV)

in welcher

$R^2$ und $Y^{3'}$ die oben angegebene Bedeutung haben,

mit Verbindungen der allgemeinen Formel (V)

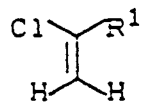

(V)

in welcher

$R^1$ die oben angegebene Bedeutung hat, umsetzt,

B) oder daß man zum Erhalt von 2-Arylpyrrolen der Formel (IIb)

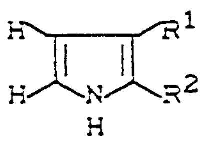

(IIb)

in welcher

$R^1$ und $R^2$ die oben angegebene Bedeutung haben,

entweder

$B_1$) N-Formylarylglycine der allgemeinen Formel (VI)

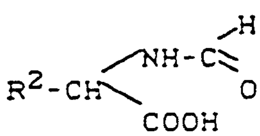

(VI)

in welcher

$R^2$ die oben angegebene Bedeutung hat,

mit Verbindungen der allgemeinen Formel (V)

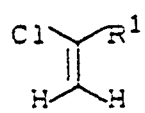

(V)

in welcher

$R^1$ die oben angegebene Bedeutung besitzt, umsetzt

oder

$B_2$) Verbindungen der allgemeinen Formel (VII)

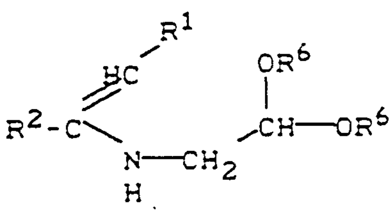

(VII)

in welcher

$R^1$ und $R^2$ die oben angegebene Bedeutung haben und

$R^6$ für Alkyl steht,
mit organischen oder anorganischen Säuren cyclisiert.

Europäisches Patentamt

# EUROPÄISCHER RECHERCHENBERICHT

Nummer der Anmeldung

EP 92 10 8332

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
| --- | --- | --- | --- |
| A | EP-A-0 310 558 (CIBA-GEIGY)<br>* Seite 18; Tabelle 4 *<br>--- | 1-10 | C07D405/04<br>C07D207/34<br>A01N43/36<br>A01N43/32 |
| A | EP-A-0 300 307 (BAYER) 25. Januar 1989<br>* das ganze Dokument *<br>----- | 1-10 | |

RECHERCHIERTE SACHGEBIETE (Int. Cl.5 )

C07D
A01N

Der vorliegende Recherchenbericht wurde für alle Patentansprüche erstellt

| Recherchenort | Abschlußdatum der Recherche | Prüfer |
| --- | --- | --- |
| DEN HAAG | 10 JULI 1992 | BERND KISSLER |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)